# EUROPEAN PATENT APPLICATION

(11) **EP 2 492 347 A1**
(43) Date of publication of application: **29.08.2012**
(21) Application number: 12382188.6
(22) Date of filing: 22.05.2012
(51) Int. Cl.: C12N 15/864

(54) **Methods for the production of vectors**

(71) Applicant: Laboratorios del. Dr. Esteve, S.A., 08041 Barcelona (ES); Universitat Autònoma De Barcelona, 08193 Barcelona (ES)
(72) Inventor: The designation of the inventor has not yet been filed
(74) Representative: Pons Ariño, Angel

(57) **Abstract**

The present invention provides methods for the production of recombinant adeno-associated viral vectors (rAAV) which comprise a nucleotide sequence having at least 85% sequence identity to SEQ ID NO: 1 that codifies for the protein SEQ ID NO: 2. The rAAVs obtained by the methods of the invention are useful for the treatment of diseases and specially, for the treatment of mucopolysaccharidoses.

## Description

### FIELD OF THE INVENTION

The present invention relates to methods for the production of vectors useful for gene therapy. The present invention relates also to the vectors obtained by the said methods, useful for the treatment of mucopolysaccharidoses (MPS), and in particular, for the treatment of mucopolysaccharidoses type III or Sanfilippo syndrome.

### BACKGROUND OF THE INVENTION

Recombinant adeno-associated viral vectors (rAAVs) have emerged as one of the vectors of choice for gene transfer applications because of their many desirable properties. AAV vectors have an excellent safety profile, as they are poorly immunogenic and have not been associated with any disease in humans. In addition, AAV vectors transduce efficiently post-mitotic cells; several preclinical studies have demonstrated that AAV vector-mediated gene transfer results in long-term gene expression in a number of animal models. See Sondhi D, et al., Gene Ther. 2005; 12:1618-1632, Wang L, et al., Blood 2005; 105:3079-3086, Donsante A, et al., J. Inherit. Metab. Dis. 2007; 30:227-238, Glenn P, et al., Blood 2009; 113:797-806, Hemsley K, et al., Mol. Genet. Metab. 2009; doi:10.1016/j.ymgme.2009.07.013, 1-10, McCarty D, et al., Gene Ther. 2009; doi:1 0.1 038/gt.2009.85, 1-13, Pañeda A, et al., Hum. Gene Ther. 2009; 20:908-917, Richard M, et al., Gene Ther. 2009; doi:10.1038/gt.2009.36, 1-11, and Pleger S, et al., Science Trans. Med. 2011; 3(92): 92ra64, 1-10. More recently, the safety and efficacy of AAV gene transfer in humans have also being demonstrated with encouraging results obtained in the treatment of various liver, muscle, central nervous system (CNS), and blood diseases. See Grimm D, et al., Hum. Gene Ther. 1999; 10:2445-2450, Flotte T, Gene Ther. 2004; 11, 805-810, Manno C, et al., Nat. Med. 2006; 12(3):342-347, LoDuca P, et al., Curr. Gene Ther. 2009; 9:104-114, and Nathwani A, et al., N. Eng. J. Med. 2011; doi:10.1056/NEJMoa1108046, 1-9.

The performance of additional pre-clinical and clinical trials with rAAV based gene therapy is limited by vector production constraints. These tests require the large scale production of highly pure and effective rAAVs, which continues to be at present a challenge in the gene therapy field. *See* Kotin R, Hum. Mol. Gen. 2011; 20(1):R2-R6.

AAV viruses belong to the *Parvoviridae* family and are characterized by a small protein capsid of 20nm containing a single stranded DNA genome of about 4.7kb. AAVs are also referred as helper-dependent viruses, or dependovirus, based on their inability to replicate in the absence of a helper virus. Humans are a natural host for wild type AAVs, but no known pathology has been associated to AAV infection in humans. Most of the early studies on the biology of wild type AAV focused on the first serotype isolated, AAV serotype 2. Wild-type AAV has the ability to integrate into a specific region of chromosome 19 in the human genome; this propensity is not maintained in rAAV because of the substitution of the viral genome with the transgene expression cassette. The structure and genome of wild type AAV is very similar for all serotypes. The genome of AAV2 contains two open reading frames (*orf*), which encode four regulatory proteins (Rep) and three structural/capsid proteins (Cap). The viral genome is flanked by two inverted terminal repeats (ITR), which are base-paired hairpin structures of 145 nucleotides length. The ITRs contain the only regulatory *cis* acting sequences required by the virus to complete its replication cycle, namely the origin of DNA replication, the terminal resolution site, and the packaging and integration signals. Two promoters, p5 and p19, regulate the expression of the *rep* gene, producing two transcripts that undergo alternative splicing to yield four Rep proteins. The Rep proteins are necessary for the viral DNA replication and integration. Rep 78 and Rep 68 are derived from the p5 promoter transcripts, whereas Rep 52 and Rep 40 are products of p19 promoter transcripts. The two major Rep proteins Rep 78 and Rep 68 are involved in viral genome excision, rescue, replication and integration, and also regulate gene expression; whereas minor Rep proteins Rep 52 and Rep 40 are involved in replicated single stranded DNA (ssDNA) accumulation and packaging. The *cap* gene encodes for the three AAV structural proteins, VP1, VP2, and VP3. *See* Kotin, 2011, *supra.*

AAVs are dependent on the presence of a helper virus for their replication and gene expression. Several viruses have been shown to support AAV production, namely adenoviruses (Ad), herpes simplex virus (HSV), vaccinia virus, and cytomegalovirus (CMV). Upon infection of a target cell, the AAV ssDNA genome is converted into double stranded DNA (dsDNA) in the nucleus in order to be transcriptionally active and start gene expression. The conversion of ssDNA to dsDNA has been identified as the limiting step for efficient gene transfer using recombinant AAV. In the absence of helper viruses, the wild type AAV (wtAAV) genome is able to integrate into the host genome and latently persist in a proviral form. These proviruses may be activated and "rescued" upon subsequent helper virus infection by inducing active replication of the genome, capsid proteins synthesis and DNA packaging, these steps taking place inside the nucleus. One feature of AAV cycle that has important consequences for rAAV production is the fact that AAV does not possess lytic capability and release of virions relies on the effect of the helper virus. See Kotin, 2011, *supra.*

The production of rAAV of different serotypes, both at small and large scales, can be performed by different methods, which can be summarized in transfection methods, methods which use producer and packaging cell lines, HSV-based methods and baculovirus-based methods. *See* Ayuso E, et al., Curr. Gene Ther. 2010; 10(6):423-436, Clément N, et al., Hum. Gene Ther. 2009; 20:796-806, Cecchini S, et al., Gen. Ther. 2008; 15:823-830, Zhang H, et al., Hum. Gene Ther. 2009; 20:922-929, and Thorne B, et al., Hum. Gene Ther. 2009; 20:707-714.

Mucopolysaccharidosis type IIIA (MPSIIIA) or Sanfilippo syndrome, is an inherited lysosomal storage disease caused by deficiency of sulfamidase, resulting in accumulation of the glycosaminoglycan (GAG) heparan sulfate. Symptoms of MPSIIIA occur in the first years of life, and are characterized by severe neurodegeneration that leads to deep mental retardation, aggressiveness, hyperactivity, and sleep alterations. Patients progressively lose the capacity of speech, swallow, and basic motor coordination. In addition to the neurological symptoms, MPSIIIA patients suffer non-neurological alterations, including hepato- and splenomegaly, skeletal and joint malformations, as well as frequent diarrhoea and respiratory tract infections. The progressive worsening of symptoms results in the death of the patient during adolescence. See Neufeld E, Muenzer J, The mucopolysaccharidoses, Scriver C, et al. Eds., "The metabolic and molecular basis of inherited disease", 8th. Ed. (McGraw-Hill Publishing Co. New York, NY, US, 2001, pp. 3421-3452).

The ability of AAV vector-mediated genetic modification of either skeletal muscle or liver to revert the established disease phenotype of 2-month-old MPSIIIA male and female mice has been reported. See Ruzo A, et al., Mol. Ther. 2011; doi:10.1038/mt.2011.220. Intramuscular administration of AAV-sulfamidase failed to achieve significant therapeutic benefit in either gender, whereas AAV8-mediated liver-directed gene transfer achieved high and sustained levels of circulating active sulfamidase, which reached normal levels in females and was fourfold higher in males, and completely corrected lysosomal GAG accumulation in most somatic tissues. Moreover, they showed how liver-directed AAV gene transfer can reverse somatic and ameliorate neurological pathology in MPSIIIA.

### SUMMARY OF THE INVENTION

The present invention provides methods to generate AAV vectors for the treatment of mucopolysaccharidoses (MPS). Therefore, the first aspect of the invention refers to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) infecting an insect cell line with either 1, 2 or 3 baculovirus expression vectors comprising the *rep* and cap genes and a nucleotide sequence having at least 85% sequence identity to SEQ ID NO: 1 that codifies for the protein SEQ ID NO: 2 interposed between a first AAV terminal repeat and a second AAV terminal repeat, and
(ii) purifying the rAAVs produced by the cell culture of step (i).

In a second aspect, the invention relates to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) transfecting competent cells with a first vector comprising a nucleotide sequence having at least 85% sequence identity to SEQ ID NO: 1 that codifies for the protein SEQ ID NO: 2 interposed between a first AAV terminal repeat and a second AAV terminal repeat; and a second vector comprising either:
   (i1) an AAV rep gene and an AAV cap gene, or
   (i2) an AAV rep gene and an AAV cap gene and adenovirus helper function,
(ii) transfecting the cells of step (i1) with either:
   (ii1) a third vector comprising the adenovirus helper function, or
   (ii2) infecting said cells with a helper virus,
(iii) culturing the transfected cell of step (i), and
(iv) purifying the rAAVs produced by the cell culture of step (ii).

In a third aspect, the invention relates to a producer cell line which integrates in its genome an AAV rep gene, an AAV cap gene, and a nucleotide sequence having at least 85% sequence identity to SEQ ID NO: 1 that codifies for the protein SEQ ID NO: 2 interposed between a first AAV terminal repeat and a second AAV terminal repeat.

In a fourth aspect, the invention relates to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) culturing the producer cell line of the third aspect of the invention,
(ii) infecting the cell line of step (i) with a helper virus, and
(iii) purifying the rAAVs produced by the cell culture of step (ii).

In a fifth aspect, the invention relates to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) culturing a packaging cell line which integrates in its genome an AAV rep gene and an AAV cap gene,
(ii) infecting the cell line of step (i) with an adenovirus comprising a nucleotide sequence having at least 85% sequence identity to SEQ ID NO: 1 that codifies for the protein SEQ ID NO: 2 interposed between a first AAV terminal repeat and a second AAV terminal repeat and with a helper virus, and
(iii) purifying the rAAVs produced by the cell culture of step (ii).

Another aspect of the present invention refers to the rAAVs obtained by any one of the methods of the present invention.

### BRIEF DESCRIPTION OF THE DRAWINGS

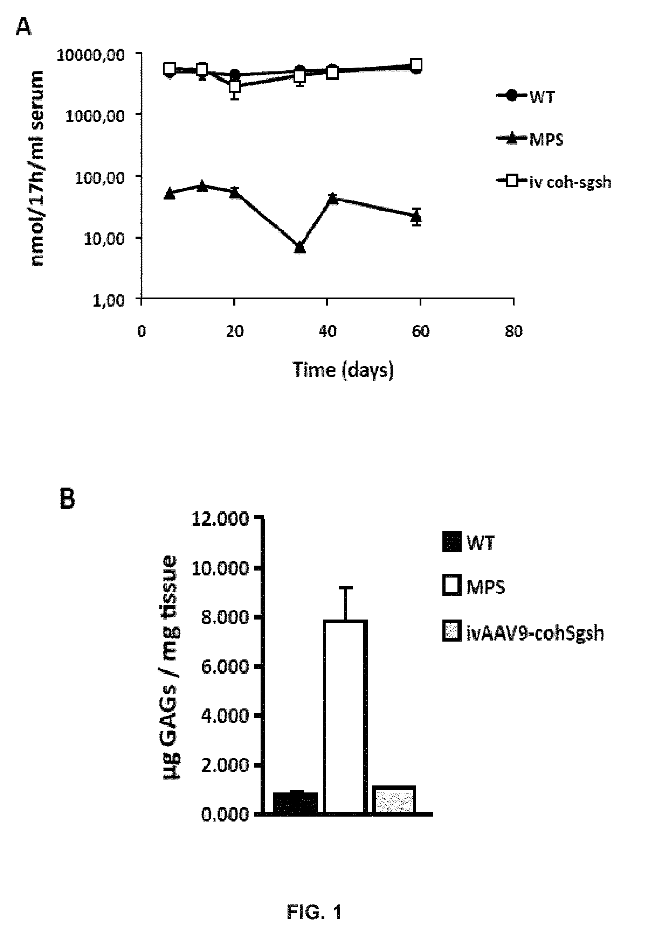

FIG. 1. Serum sulfamidase activity and liver GAG content in intravenous AAV9-co-hu-SFMD injected animals. (A) Sulfamidase activity in the serum measured with a fluorogenic substrate. (B) GAG storage in the liver 2 months after vector administration.

### DEFINITIONS

The term "% sequence identity", as used herein, refers to the percentage of nucleotides of a candidate sequence that are identical to the nucleotides in SEQ ID NO: 1, after aligning the sequences to achieve the maximum % sequence identity. The % sequence identity can be determined by any methods or algorithms established in the art, such as ALIGN or BLAST. Herein, the % sequence identity is calculated dividing the number of nucleotides that are identical after aligning SEQ ID NO: 1 and the candidate sequence, by the total number of nucleotides in SEQ ID NO: 1 and multiplying the result by 100. As used herein, "% sequence identity" is determined by comparing two optimally aligned sequences over a comparison window, where the fragment of the sequence in the comparison window may comprise additions or deletions (e.g. gaps or overhangs) as compared to the reference sequence (which does not comprise additions or deletions) for optimal alignment of the two sequences. The percentage is calculated by determining the number of positions at which the identical amino acid residue or nucleic acid residue occurs in both sequences to yield the number of matched positions, dividing the number of matched positions by the total number of positions in the comparison window and multiplying the result by 100 to provide the percentage of sequence identity. Algorithms to align sequences are known in the art. Optimal alignment of sequences for comparison can be conducted, for instance, by the Smith-Waterman local homology algorithm, by the Needleman-Wunsch homology alignment algorithm, by the Pearson-Lipman similarity search method, by computerized implementations of these algorithms or by manual alignment and visual inspection. See Smith T, Waterman M, Adv. Appl. Math. 1981; 2:482-489; Needleman S, Wunsch C, J. Mol. Biol. 1970; 48:443-453; Pearson W, Lipman D, Proc. Natl. Acad. Sci. USA 1988; 85:2444-2448; Tatusova T, Madden T, FEMS Microbiol. Lett. 1999; 174:247-250; the GAP, BESTFIT, FASTA and TFASTA programs, Wisconsin Genetics Software Package, Genetics Computer Group, Madison, WI, US; Ausubel F, et al., Eds., "Short Protocols in Molecular Biology", 4th Ed. (John Wiley and Sons, Inc., New York, NY, US, 1997).

The expression "adenovirus helper function", as used herein, refers to the sequences needed to provide said helper function, which comprises virus associated-RNA, E1A, E1B, E2A and E4 genes. See Chang L, et al., J. Virol. 1989; 63:3479-3488, Pilder S, et al., J. Virol. 1984; 52:664-671, Querido E, et al., J. Virol. 1997; 71:3788-3798, Steegenga W, et al., Oncogene 1998; 16:349-357, Carter B, et al., Virology 1992; 191:473-476, Chang L, et al., J. Virol. 1990; 64:2103-2109, Ward P, et al., J. Virol. 1998; 72:420-427, and West M, et al., Virology 1987; 160:38-47. The vectors or plasmids with adenovirus helper function are designed to carry only the subset of adenoviral genes that is essential for rAAV production and thus the plasmids are noninfectious (i.e. cells transfected with these constructs do not produce infectious helper virus). The helper plasmid preferably lacks the AAV-2 p5 promoter, including its Rep protein-binding site, to make sure that no replication competent AAvs are generated.

The term "codify", as used herein, refers to the genetic code that determines how a nucleotide sequence is translated into a polypeptide or a protein. The order of the nucleotides in a sequence determines the order of amino acids along a polypeptide or a protein.

The term "comprising" or "comprises", as used herein, discloses also "consisting of" according to the generally accepted patent practice.

The term "individual", as used herein, refers to an arbitrary animal, preferably, a human or non-human mammal, more preferably, a mouse, rat, other rodents, rabbit, dog, cat, pig, cow, horse or primate, more preferably further, a human being.

The term "nucleotide sequence", as used herein, refers to a nucleic acid molecule, either DNA or RNA, containing deoxyribonucleotides or ribonucleotides. The nucleic acid may be double stranded, single stranded, or contain portions of both double stranded or single stranded sequence.

The term "operably linked", as used herein, is intended to mean that the nucleotide sequence of interest is linked to the regulatory sequence(s) in a manner that allows for expression of the nucleotide sequence (e.g. in an in vitro transcription/translation system or in a host cell when the vector is introduced into the host cell). See Auer H, Nature Biotechnol. 2006; 24: 41-43.

The term "protein", as used herein, refers to a linear chain of amino acids. Proteins can suffer post-translational modifications, like the conversion of a cysteine residue to 3-oxoalanine, glycosylation or metal binding. Glycosilation of a protein is the addition of different carbohydrates that are linked covalently to the amino acid chain.

The term "vector genome (vg)" and the term "viral genome" are used interchangeably and refer to the number of particles containing the vg. Vector genome units per kg of body weight (vg/kg) is the most commonly unit adopted for dose determination in gene transfer.

### DETAILED DESCRIPTION OF THE INVENTION

High amounts of recombinant adeno-associated viral vectors (rAAV) are needed for the treatment of human patients, ranging from 10¹³ to 10¹⁷ viral genomes. The present invention provides methods for the production of rAAVs, including purification and characterization, and suitable for large-scale production of rAAVs for the treatment of disease, preferably for the treatment of mucopolysaccharidoses, and in particular, for the treatment of mucopolysaccharidoses type III or Sanfilippo syndrome. These methods employ different technologies, such as baculovirus and insect cells, transient double or triple transfection methods, producer/packaging cell lines and helper vectors like Herpesvirus-based systems. Moreover, the present invention provides methods for the production of rAAVs compliant with current Good Manufacturing Practice (cGMP) and scalable for industrial production.

Therefore, a first aspect of the present invention refers to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) infecting an insect cell line with either 1, 2 or 3 baculovirus expression vectors comprising the *rep* and cap genes and a nucleotide sequence having at least 85% sequence identity to SEQ ID NO: 1 that codifies for the protein SEQ ID NO: 2 interposed between a first AAV terminal repeat and a second AAV terminal repeat, and
(ii) purifying the rAAVs produced by the cell culture of step (i).

In a preferred embodiment of the first aspect of the present invention, the nucleotide sequence has at least 90% sequence identity to SEQ ID NO: 1. Preferably, it has 95% sequence identity to SEQ ID NO: 1, more preferably it has 98% sequence identity to SEQ ID NO: 1, much more preferably, it has 99% sequence identity to SEQ ID NO: 1. In a preferred embodiment of the first aspect of the present invention, the nucleotide sequence having at least 85% sequence identity to SEQ ID NO: 1 that codifies for the protein SEQ ID NO: 2, is SEQ ID NO: 1. In a preferred embodiment, the nucleotide sequence is operably linked to a CAG or a hAAT promoter. Preferably, the nucleotide sequence is SEQ ID NO: 1 and it is operably linked to a CAG promoter. In a preferred embodiment of the first aspect of the present invention, the sequence of the hAAT promoter is SEQ ID NO: 3.

The expression cassette comprising a nucleotide sequence having at least 85% sequence identity to SEQ ID NO: 1 that codifies for the protein SEQ ID NO: 2, wherein a CAG or hAAT promoter is operably linked to said nucleotide sequence and is cloned between the AAV ITRs.

In a preferred embodiment of the first aspect of the present invention, the insect cell line is Sf9 cell line. Preferably, the insect cell line is stably transfected with the *rep* and cap genes. More preferably, the insect cell line is infected with 1 baculovirus expression vector comprising the nucleotide sequence having at least 85% sequence identity to SEQ ID NO: 1 that codifies for the protein SEQ ID NO: 2 interposed between a first AAV terminal repeat and a second AAV terminal repeat, and an inmediate-early 1 transcriptional transregulator. Preferably, the inmediate-early 1 transcriptional transregulator is the *ie-1* gene of the baculovirus *Autographa californica* nuclear polyhedrosis virus (AcMNPV), which encodes the transregulatory protein IE1.

In another preferred embodiment of the first aspect of the present invention, the insect cell line is infected with 2 baculovirus expression vectors, one comprising the *rep* and *cap* genes, and the second comprising the nucleotide sequence having at least 85% sequence identity to SEQ ID NO: 1 that codifies for the protein SEQ ID NO: 2 interposed between a first AAV terminal repeat and a second AAV terminal repeat.

In another preferred embodiment of the first aspect of the present invention, the insect cell line is infected with 3 baculovirus expression vectors, one comprising the *rep* genes, the second comprising the *cap* genes, and the third one comprising the nucleotide sequence having at least 85% sequence identity to SEQ ID NO: 1 that codifies for the protein SEQ ID NO: 2 interposed between a first AAV terminal repeat and a second AAV terminal repeat.

In a preferred embodiment of the present invention, the serotype of the rAAVs is 1, 2, 5, 7, 8 or 9. Preferably, the serotype of the rAAVs is 9.

In a preferred embodiment of the first aspect of the present invention, the *rep* genes are Rep78, Rep68, Rep52 and Rep40. Preferably, the expression of Rep78 is directed by an attenuated baculovirus promoter and/or the expression of Rep52 is directed by a strong late baculovirus promoter, preferably by p10. More preferably, a combination of the Rep52 and Rep 78 genes is utilized.

In a preferred embodiment of the present invention, 10¹³ to 10¹⁵ particles of rAAV are obtained per liter of culture.

Another aspect of the present invention refers to the rAAVs obtained by any one of the methods of the present invention, preferably obtained by the first method of the invention. In a preferred embodiment, the rAVVs are used for gene therapy. Preferably, the gene therapy is for the treatment of mucopolysaccharidoses, more preferably of mucopolysaccharidosis type III or Sanfilippo syndrome. Therefore, another aspect of the present invention is a pharmaceutical composition comprising a therapeutically effective amount of the rAAVs of the invention. Preferably, the pharmaceutical composition is administered by parenteral administration, preferably for intravenous or intracisternal administration.

In a preferred embodiment, the rAAVs of the invention or the pharmaceutical composition comprising said rAAVs are used for increasing the sulfamidase activity in the body.

In a second aspect, the present invention refers to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) transfecting competent cells with a first vector comprising a nucleotide sequence having at least 85% sequence identity to SEQ ID NO: 1 that codifies for the protein SEQ ID NO: 2 interposed between a first AAV terminal repeat and a second AAV terminal repeat; and a second vector comprising either:
   (i1) an AAV rep gene and an AAV cap gene, or
   (i2) an AAV rep gene and an AAV cap gene and adenovirus helper function,
(ii) transfecting the cells of step (i1) with either:
   (ii1) a third vector comprising the adenovirus helper function, or
   (ii2) infecting said cells with a helper virus,
(iii) culturing the transfected cell of step (i), and
(iv) purifying the rAAVs produced by the cell culture of step (ii).

In a preferred embodiment of the second aspect of the invention, the method comprises the following steps:
(i) transfecting competent cells with a first vector comprising a nucleotide sequence having at least 85% sequence identity to SEQ ID NO: 1 that codifies for the protein SEQ ID NO: 2 interposed between a first AAV terminal repeat and a second AAV terminal repeat; and a second vector comprising an AAV rep gene and an AAV cap gene;
(ii) transfecting the cells of step (i) with a third vector comprising the adenovirus helper function;
(iii) culturing the transfected cell of step (i); and
(iv) purifying the rAAVs produced by the cell culture of step (ii).

In a preferred embodiment of the second aspect of the invention, the method comprises the following steps:
(i) transfecting competent cells with a first vector comprising a nucleotide sequence having at least 85% sequence identity to SEQ ID NO: 1 that codifies for the protein SEQ ID NO: 2 interposed between a first AAV terminal repeat and a second AAV terminal repeat; and a second vector comprising an AAV rep gene and an AAV cap gene and adenovirus helper function,
(ii) culturing the transfected cell of step (i), and
(iii) purifying the rAAVs produced by the cell culture of step (ii).

In a preferred embodiment of the second aspect of the invention, the method comprises the following steps:
(i) transfecting competent cells with a first vector comprising a nucleotide sequence having at least 85% sequence identity to SEQ ID NO: 1 that codifies for the protein SEQ ID NO: 2 interposed between a first AAV terminal repeat and a second AAV terminal repeat; and a second vector comprising an AAV rep gene and an AAV cap gene;
(ii) infecting said cells with a helper virus,
(iii) culturing the transfected cell of step (i), and
(iv) purifying the rAAVs produced by the cell culture of step (ii).

In a preferred embodiment of the present invention, the nucleotide sequence is SEQ ID NO: 1. Preferably, the nucleotide sequence is operably linked to a CAG or a hAAT promoter. More preferably, the nucleotide sequence is SEQ ID NO: 1 and it is operably linked to a CAG promoter.

The preferred competent cells when the method of the second aspect of the invention comprises steps (i), (i1) and (ii1) are HEK 293 cells which have been transformed with an adenovirus of type 5 to stably express the E1A and E1B genes.

The transfection may be carried out by any of the methods or protocols known in the state of the art, like for example, but not limited to, calcium phosphate, polycations such as polyethylenimine (PEI), electroporation, or lipid transfection See Wright J, Hum. Gene Ther. 2009; 20:698-706; Brown T, "Gene Cloning" (Chapman & Hall, London, GB, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, GB, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

The helper virus is preferably an adenovirus, preferably a type 5 adenovirus (Ad5). Ad5 can be easily produced in suspension culture in an animal-derived component-free (ADCF) process with the same lineage of HeLa S3 cells as can be used to generate the producer cell line. It is also easily frozen and stored for many years at -70°C without loss of infectivity and can be concentrated sufficiently to allow a small-volume inoculum. The helper virus provides functional E1 genes but is conditionally replication defective.

In a third aspect, the present invention refers to a producer cell line which integrates in its genome an AAV rep gene, an AAV cap gene, and a nucleotide sequence having at least 85% sequence identity to SEQ ID NO: 1 that codifies for the protein SEQ ID NO: 2 interposed between a first AAV terminal repeat and a second AAV terminal repeat.

In a preferred embodiment of the third aspect of the invention, the producer cell line is capable of growing in suspension. Preferably, rep genes expression is controlled to avoid its overexpression. Preferably, the producer cell line does not contain the adenovirus E1 gene. The cell line may be designed to regulate rep transcription by inserting termination sequences into the coding region of Rep proteins, which can be removed by the infection with an adenovirus comprising Cre.

In a preferred embodiment of the third aspect of the invention, the producer cell line is HeLa, A549, Vero (derived from the African green monkey) or HEK293, preferably HeLa or A549.

In a fourth aspect, the present invention refers to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) culturing the producer cell line of the third aspect of the invention,
(ii) infecting the cell line of step (i) with a helper virus, and
(iii) purifying the rAAVs produced by the cell culture of step (ii).

In a fifth aspect, the present invention refers to a method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) culturing a packaging cell line which integrates in its genome an AAV rep gene and an AAV cap gene,
(ii) infecting the cell line of step (i) with an adenovirus comprising a nucleotide sequence having at least 85% sequence identity to SEQ ID NO: 1 that codifies for the protein SEQ ID NO: 2 interposed between a first AAV terminal repeat and a second AAV terminal repeat and with a helper virus, and
(iii) purifying the rAAVs produced by the cell culture of step (ii).

In a preferred embodiment of the fifth aspect of the invention, the packaging cell line is HeLa, A549, Vero (derived from the African green monkey) or HEK293, or is derived from any of said cell lines, as for example, C12 cell line, K209 cell line or B-50 cell line.

The producer cell line is preferably obtained as described previously in the art. See Thorne, 2009, *supra.* The rAAV manufacturing process based on a producer cell line comprises the following steps: first, cells are thawed from the master cell bank and expanded in a virus-free area. Next, vector production is induced by infection with Ad5, which also becomes a byproduct in this process. After production, a series of product recovery steps are performed. These steps have the added advantage of restricting most of the helper virus to the production area. Finally, a purification stage removes process impurities and includes sufficient orthogonal mechanisms of viral clearance; thus providing a wide safety margin of Ad5-free final bulk product. The manufacturing process begins with thaw and expansion of a cGMP cell bank in an Ad5-free cell culture room. All steps are performed in suspension culture in single-use vessels, ranging from shake flasks to a 10-liter rocking bioreactor. The final stage of cell expansion is performed in a 50-liter single-use stirred tank perfusion bioreactor in the production room. Production occurs in a 250-liter single-use stirred tank bioreactor and is initiated by infection with wild-type Ad5. At the end of production, vector product is recovered from the cell culture supernatant fluid in a series of steps designed to remove cells, free DNA, and Ad5, containing these process components within the production room. These include depth filtration, nuclease digestion, and flow-through ion exchange (FT-IEX). To provide a convenient optional hold step before purification, recovery ends with concentration by tangential flow ultrafiltration (UF-TFF). Product is then transferred to a separate purification room for chromatographic purification and two additional viral clearance steps: heat inactivation and nanofiltration. Additional segregation is used for operations that follow nanofiltration. After the final polishing column, buffer exchange by ultrafiltration/diafiltration for formulation results in final purified bulk vector. Only individual recovery and purification steps are AAV serotype dependent. Adenovirus and AAVs have different thermostabilities. For example, a 30 minutes incubation of the rAAV product at 56-60°C inactivates contaminating adenovirus effectively, while rAAV remains infectious. The inactivation of the helper virus is essential and the well-characterized different thermostabilities provide good Ad5 clearance by heat inactivation, which also does not need to remove anything to terminate the inactivation procedure, as does the use of detergents. "Nanofiltration" is an additional viral clearance measure positioned near the end of the purification process to minimize the potential for filter fouling. For producing the purified bulk drug substance, a final step of buffer exchange by diafiltration/ultrafiltration for formulation is performed.

In a preferred embodiment of the invention, the helper virus comprises the Ad genes E1A and E1B, E2A, VA, and E4orf6. In another preferred embodiment of the invention, the helper virus does not comprise the E1 open reading frame (ORF), which is provided by the producer cell line.

In a preferred embodiment of the invention, the helper virus is a Herpes Simplex Virus type 1 (HSV). In a preferred embodiment of the invention, the vector comprising the adenovirus helper function is based on HSV. Preferably, the HSV is replication defective, as mutant *d27.1.* In another preferred embodiment of the invention, the HSV is a disabled, single-cycle (DISC) herpesvirus, lacking expression of glycoprotein H and therefore increasing the safety of the system.

In another preferred embodiment of the invention, the HSV is a recombinant HSV (rHSV). Preferably, the *rep* and cap genes of AAV are inserted into the thymidine kinase (TK) locus of a replication defective HSV by homologous recombination. The rHSV helper vectors of the invention can be produced in bioreactors using cell in suspension, and the AAV serotype can be easily adapted. Preferably, rHSV are propagated in Vero or baby hamster kidney (BHK) cells. The rHSV-based system of the present invention is capable of supporting large-scale production of rAAV batches, generating more than 2x10¹⁵ DNA-containing particles from a single 10-liter culture run.

In a preferred embodiment of the present invention, the helper virus is an rHSV, the production cell line is 293 or BHK, and said production cell line is co-infected with rHSV comprising a nucleotide sequence having at least 85% sequence identity to SEQ ID NO: 1 that codifies for the protein SEQ ID NO: 2 interposed between a first AAV terminal repeat and a second AAV terminal repeat, and rHSV with *rep*/*cap* genes.

In a preferred embodiment of the present invention, the rAAV production method employs a *rep*/*cap* cell line and an Ad/AAv hybrid infection, resulting in a 5- to 10- fold increase in vector yield when compared with the triple transfection method.

The method of the first aspect of the present invention uses strong promoters and therefore allows producing high quantities of rAAVs. The scale up is performed by growing insect cells in suspension in a bioreactor of different volumes. The purification of the rAAVs of the present invention is benefited from the fact that it is a serum free system.

In a preferred embodiment of the present invention, at a small scale production, the baculovirus-system produced rAAVs are recovered from the Sf9 insect cells (both the intra- and extracellular vectors) by freeze-thaw lysis of the culture directly in the flask. The cell lysate may be nuclease-treated to reduce viscosity and digest extravirion DNA. Centrifugation or filtration may be used to remove debris. To concentrate the rAAV, polyethylene glycol (PEG, 8000 MW) is added to the clarified lysate (2%, w/v) to precipitate the rAAV particles. The precipitated rAAV can be re-suspended in the appropriate medium for subsequent processing.

In a preferred embodiment of the present invention, at a large scale production in a bioreactor of around 40 to 500 liters, insect cells are grown at around 27-28°C in serum-free media and agitation, with controlled oxygen concentration, pH and dielectric permittivity

In a preferred embodiment of the present invention, the purification of the rAAVs of the invention may comprise the steps of cell lysis; clarification (by high-speed centrifugation or microfiltration, or PEG-based precipitation); gradient centrifugation (CsCl or iodixanol gradients); and column chromatography alone or in combination with gradient centrifugation.

At a large scale, cell lysis can be performed by chemical lysis or mechanical lysis, or a combination of both, which can be followed by nuclease treatment and filtration.

In a preferred embodiment, surfactants such as deoxycholate or Triton X-100 are used for cell disruption and rAAV recovery. Also, high flow rate homogenizers can be used for mechanical lyses of the cells at large scale production of rAAVs.

At large scale production, in order to reduce the liquid volume of the clarified cell lysate, tangential flow filtration using hollow fiber or flat cassettes can be used. The tangential flow filtration has the advantage that a large amount of low molecular mass cell proteins are removed, especially when coupled with diafiltration.

In a preferred embodiment, the purification or rAAVs is performed by column chromatography alone or in combination with gradient centrifugation. Immunoaffinity columns can also be used.

Purification of rAAVs may also be performed by methods based on differential CHCl₃ denaturation and solubility.

In a preferred embodiment, the purification at large scale of rAAVs comprises ion-exchange chromatography, where the external charge of the capsid is used to discriminate empty capsids from genome-containing AAVs.

In a preferred embodiment, the purification of the rAAVs comprises a combination of chromatography and gradient ultracentrifugation, where firstly all particles, full and empty, are recovered by applying the clarified cell lysate onto an ion-exchange chromatography column, and secondly a CsCl gradient ultracentrifugation is used to eliminate impurities like empty capsids.

In a preferred embodiment of the present invention, the purification of the rAAVs is performed by a closed two-column chromatography system of a cationic exchange chromatography to capture rAAV virions, followed by a polishing step with anionic exchange chromatography. This purification system results in highly efficient recovery of rAAVs from crude lysates with substantially improved purity and potency, and reduced immunity issues.

In a preferred embodiment, the methods of the present invention comprise steps for reducing impurities such as residual proteins, nucleic acids and especially empty capsids. The use of benzoase or other methods for nucleic acid digestion to eliminate the non-encapsidated nucleic acids from cell lysis and production plasmids is preferred.

The characterization of the final rAAVs obtained by the methods of the present invention may be performed by any of the potency, purity, integrity and sterility determination methods known in the art. Determining particle dispersion in solution by using suitable means, such as dynamic light scattering, is especially important when preparing the rAAV formulation.

The final vector formulation may comprise adequate excipients to guarantee vector stability, or surfactants than avoid the non-specific binding of AAV particles to surfaces, as for example to the administration device. Also, excipients, surfactants, or other additives, may be used to control the loss of activity due to particle aggregation, proteolysis or oxidation.

Vector titration may be performed by DNA-based protocols by hybridization (as dot-blot) or quantitative PCR, where the rAAV stocks have been previously digested with DNAase, by capsid quantification protocols like electronic microscopy, antibody-based ELISA or calculation of the protein concentration, or by infection or transduction titers. The ratio of viral particles able to successfully infect vs. total virus titer may be measured, for example, but not limited to, by using cell lines expressing *rep*/*cap* genes and measuring the number of viral genomes by quantitative PCR. The transduction titer may be measured using rAAVs which comprise a reporter gene.

Electron microscopy provides useful information about the capsid morphology and corroborates other results regarding aggregation, concentration, and empty versus filled capsids.

The rAAVs produced by the methods of the present invention are submitted to vector quality controls of sterility, endotoxin, mycoplasma, adventitious viruses, empty capsids, replication competent AAV (rcAAV), encapsidated DNA impurities, elimination of helper viruses, protein impurities.

In a preferred embodiment of the present invention, stuffer DNA is included in the vector to increase the size of the backbone and therefore reduce the packaging of undesired DNA by reverse packaging.

The replication of competent AAV particles by homologous or non-homologous recombination is prevented by applying techniques known in the art. See Samulski R, et al., J. Virol. 1989; 63:3822-3828.

Another aspect of the present invention refers to the rAAVs obtained by any one of the methods of the present invention, preferably obtained by the first method of the invention. In a preferred embodiment, the rAVVs are used for gene therapy. Preferably, the gene therapy is for the treatment of mucopolysaccharidoses, more preferably of mucopolysaccharidosis type III or Sanfilippo syndrome. Therefore, another aspect of the present invention is a pharmaceutical composition comprising a therapeutically effective amount of the rAAVs of the invention. Preferably, the pharmaceutical composition is administered by parenteral administration, preferably for intravenous or intracisternal administration.

In a preferred embodiment, the rAAVs of the invention or the pharmaceutical composition comprising said rAAVs are used for increasing the sulfamidase activity in the body.

One embodiment of the present invention is a method for the production of rAAVs of serotype 9, wherein said rAAVs comprise a nucleotide sequence having at least 95% sequence identity to SEQ ID NO: 1 that codifies for the protein SEQ ID NO: 2, wherein the method comprises the following steps: (i) infecting an Sf9 insect cell line stably transfected with the rep and cap genes with 1 baculovirus expression vector comprising an inmediate-early 1 transcriptional transregulator and a nucleotide sequence having at least 95% sequence identity to SEQ ID NO: 1 that codifies for the protein SEQ ID NO: 2, where said nucleotide sequence is operably linked to a CAG or a hAAT promoter, interposed between a first AAV terminal repeat and a second AAV terminal repeat, and (ii) purifying the rAAVs produced by the cell culture of step (i) by combined gradient ultracentrifugation and column chromatography.

An additional embodiment of the present invention is a method for the production of rAAVs, wherein said rAAVs comprise a nucleotide sequence having at least 98% sequence identity to SEQ ID NO: 1 that codifies for the protein SEQ ID NO: 2, wherein the method comprises the following steps: (i) infecting an insect cell line with 2 baculovirus expression vectors, one comprising the rep genes Rep78 and Rep52 and cap genes and the second comprising a nucleotide sequence having at least 98% sequence identity to SEQ ID NO: 1 that codifies for the protein SEQ ID NO: 2, operably linked to a hAAT promoter, interposed between a first AAV terminal repeat and a second AAV terminal repeat, and (ii) purifying the rAAVs produced by the cell culture of step (i).

Another additional embodiment of the present invention is a method for the production of rAAVs of serotype 9, wherein said rAAVs comprise SEQ ID NO: 1, wherein the method comprises the following steps: (i) infecting an insect cell line with 3 baculovirus expression vectors (BEV), one comprising the rep genes Rep78 and Rep52 where the expression of Rep78 is directed by an attenuated baculovirus promoter and the expression of Rep52 is directed by a strong late baculovirus promoter, preferably by p10, the second BEV comprising the cap genes and the third BEV comprising SEQ ID NO: 1, operably linked to the hAAT promoter of SEQ ID NO: 3, interposed between a first AAV terminal repeat and a second AAV terminal repeat, and (ii) purifying the rAAVs produced by the cell culture of step (i).

Another additional embodiment of the present invention is a method for the production of rAAVs of serotype 9, wherein said rAAVs comprise SEQ ID NO: 1, wherein the method comprises the following steps: (i) transfecting competent cells with a first vector comprising SEQ ID NO: 1 interposed between a first AAV terminal repeat and a second AAV terminal repeat; and a second vector comprising an AAV rep gene and an AAV cap gene; (ii) transfecting the cells of step (i) with a third vector comprising the adenovirus helper function; (iii) culturing the transfected cell of step (i); and (iv) purifying the rAAVs produced by the cell culture of step (ii).

Another additional embodiment of the present invention is a method for the production of rAAVs of serotype 2, wherein said rAAVs comprise a nucleotide sequence having at least 90% sequence identity to SEQ ID NO: 1 that codifies for the protein SEQ ID NO: 2, wherein the method comprises the following steps: the following steps: (i) transfecting A549 cells with a first vector comprising a nucleotide sequence having at least 90% sequence identity to SEQ ID NO: 1 that codifies for the protein SEQ ID NO: 2 interposed between a first AAV terminal repeat and a second AAV terminal repeat; and a second vector comprising an AAV rep gene and an AAV cap gene and adenovirus helper function, (ii) culturing the transfected cell of step (i), and (iii) purifying the rAAVs produced by the cell culture of step (ii).

Another additional embodiment of the present invention is a method for the production of rAAVs of serotype 7, wherein said rAAVs comprise a nucleotide sequence having at least 99% sequence identity to SEQ ID NO: 1 that codifies for the protein SEQ ID NO: 2, wherein the method comprises the following steps: (i) transfecting HeLa cells with a first vector comprising a nucleotide sequence having at least 99% sequence identity to SEQ ID NO: 1 that codifies for the protein SEQ ID NO: 2 interposed between a first AAV terminal repeat and a second AAV terminal repeat; and a second vector comprising an AAV rep gene and an AAV cap gene; (ii) infecting said cells with a helper HSV, (iii) culturing the transfected cell of step (i), and (iv) purifying the rAAVs produced by the cell culture of step (ii).

Another additional embodiment of the present invention is a A549 producer cell line which does not contain the adenovirus E1 gene and which integrates in its genome an AAV rep gene, an AAV cap gene, and a nucleotide sequence having at least 98% sequence identity to SEQ ID NO: 1 that codifies for the protein SEQ ID NO: 2 interposed between a first AAV terminal repeat and a second AAV terminal repeat.

Another additional embodiment of the present invention is a method for the production of rAAV of serotype 9, wherein said rAAVs comprise a nucleotide sequence having at least 99% sequence identity to SEQ ID NO: 1, wherein the method comprises the following steps: (i) culturing a Vero producer cell line which does not contain the adenovirus E1 gene and which integrates in its genome an AAV *rep* gene, an AAV *cap* gene, and a nucleotide sequence having at least 98% sequence identity to SEQ ID NO: 1 interposed between a first AAV terminal repeat and a second AAV terminal repeat, (ii) infecting the cell line of step (i) with a helper virus, and (iii) purifying the rAAVs produced by the cell culture of step (ii).

Another additional embodiment of the present invention is a method for the production of rAAV of serotype 9, wherein said rAAVs comprise SEQ ID NO: 1, wherein the method comprises the following steps: (i) culturing a packaging B-50 cell line which integrates in its genome an AAV *rep* gene and an AAV *cap* gene, (ii) infecting the cell line of step (i) with an adenovirus comprising SEQ ID NO: 1 interposed between a first AAV terminal repeat and a second AAV terminal repeat and with a helper virus, and (iii) purifying the rAAVs produced by the cell culture of step (ii).

Having described the invention in general terms, it will be more easily understood by reference to the following examples which are presented as an illustration and are not intended to limit the present invention.

### Example 1

### rAAVs produced using Baculovirus

rAAVs were produced in Sf9 insect cells grown in suspension infected with three recombinant baculovirus expression vectors (BEV), where one carries the different serotypes capsid proteins, another carries the AAV Rep78 and Rep52 gene and the third one carries the AAV sulfamidase construct described above, in between the ITRs. See Cecchini, 2008, *supra.* Sf9 cells were infected in early log phase, approximately 2x10⁶ cells per ml. The process can be monitored closely to determine the optimal harvesting time of rAAVs. The dielectric permittivity is measured to estimate cell density and biomass in the cell culture, which is correlated with vector production. The rAAVs were harvested between 48 and 72 hours post infection and the yield of transducing units was around 2x10¹² per liter of cell culture. rAAVs were purified as explained below.

rAAVs were also produced in Sf9 insect cells grown in suspension infected with two recombinant baculovirus expression vectors (BEV), where one carries both the *rep* and *cap* genes and the second carries the AAV sulfamidase construct described above, in between the ITRs. The infection, harvest and rAAVs purification was done as described above. The percentage of competent cells to produce rAAVs was increased with this two baculovirus strategy.

rAAVs were also produced in stable Sf9 insect cells which incorporates integrated copies of *rep* and *cap* genes. This stable Sf9 cell line was generated as described in the art. *See* Aslanidi G, et al., Proc. Natl. Acad. Sci. USA 2009; 106(13): 5059-5064. This stable transfected Sf9 cell line is infected with a helper baculovirus expression vector carrying the AAV construct and inmediate-early (IE-1) transcriptional transregulator. The *ie-1* gene of the baculovirus *Autographa californica* nuclear polyhedrosis virus (AcMNPV) encodes a transregulatory protein (IE1) which accelerates the expression of early and late virus genes. Transcription of *ie-1* occurs immediately upon infection.

### Example 2

### rAAVs produced by transient transfection

The AAV vectors described herein were constructed by triple transfection. The materials required for making the vectors were: HEK293 cells (expressing E1 genes), helper plasmid providing adenovirus function, helper plasmid providing AAV *rep* genes from serotype 2 and cap genes from the desired serotype (i.e. AAV1, AAV2, AAV5, AAV7, AAV8, AAV9) and, finally, the backbone plasmid with ITRs and the construct of interest.

To generate sulfamidase-expressing AAV vectors, the cDNA of murine sulfamidase was cloned into an AAV backbone plasmid under the control of the ubiquitous hybrid promoter CAG (that contains the chicken β-actin promoter and CMV enhancer) or liver-specific human alpha1-antitrypsin (hAAT) promoter.

Vectors were generated by helper virus-free transfection of HEK293 cells using three plasmids with modifications. *See* Matsushita T, et al., Gene Ther. 1998; 5:938-945 and Wright J, et al., Mol. Ther. 2005; 12:171-178. Cells were cultured to 70% confluence in roller bottles (RB) (CeIIBIND®, Corning Inc., Acton, MA, US) in DMEM supplemented with 10% FBS and then co-transfected with: 1) a plasmid carrying the expression cassette flanked by the viral ITRs (described above); 2) a helper plasmid carrying the AAV rep2 and the correspondent cap (cap1 and cap9 genes; and 3) a plasmid carrying the adenovirus helper functions. rAAVs were purified as explained below.

### Example 3

### rAAVs produced using a stable producer cell line

Stable producer cell lines were obtained by transfection with the AAV construct described above, and with genes *rep2* and *cap* from different serotypes, and infected with a helper virus. As helper virus, both wild type adenovirus and thermo-sensitive variants were used. rAAVs were purified as explained below.

### Example 4

### rAAVs produced using a stable packaging cell line

Stable packaging cell lines were obtained by transfection with genes *rep2* and *cap* from different serotypes, and infected with a helper adenovirus (both wild type adenovirus and thermo-sensitive variants were used) and also infected with a first generation adenovirus lacking E1, with the AAV construct of interest described above. rAAVs were purified as explained below.

### Example 5

### rAAVs produced using Herpes virus

Replication-deficient HSV deleted for TK and ICP27 genes were used. The *rep2* and different serotypes *cap* genes were cloned in TK site of the HSV genome by homologous recombination. rHSV stocks were propagated in BHK cells in suspension. For rAAV production, 293 cells were co-infected with rHSV carrying the AAV genome described above and rHSV carrying the *rep2* and different serotypes *cap* genes. rAAVs were purified as explained below.

### Example 6

### Purified by gradient ultracentrifugation and/or column chromatography

Vectors were purified by cell lysis or cell disruption, clarification, and either gradient centrifugation or column chromatography or a combination of both. Cell lysis or cell disruption was chemical, mechanical or a combination of both. Mechanical lysis was performed by freeze-thaw lysis of the culture directly in the flask or by high flow rate homogenizers in large scale production, whereas chemical lysis was performed or by using surfactants such as deoxycholate or Triton X-100. Clarification was performed by high-speed centrifugation, microfiltration, or PEG-based precipitation. For clarification, nuclease treatment to reduce viscosity and digest extravirion DNA and filtration was performed to reduce debris. Also, benzoase was used for nucleic acid digestion to eliminate the non-encapsidated nucleic acids from cell lysis and production plasmids. To concentrate the rAAV, polyethylene glycol (PEG, 8000 MW) was added to the clarified lysate (2%, w/v) to precipitate the rAAV particles. A combination of ion exchange chromatography with CsCl gradient ultracentrifugation was used.

Vectors were also purified by two consecutives cesium chloride gradients using either a standard protocol or an optimized protocol as previously described. *See* Ayuso, 2010, *supra.* Vectors were dialyzed against PBS, filtered, titred by qPCR and stored at -80°C until use.

### Example 7

### Characterization of rAAVs

Characterization of purified rAAV stocks consisted of silver staining of SDS-PAGE separation of proteins (purity), ratio of rAAV full capsids (qPCR) to infectious particles (TCID₅₀), residual protein (ELISA), and residual DNA (qPCR). Quality control concluded that vector stocks were of high purity and potency, and were substantially free of undesired proteins, such as HSV protein, and DNA.

### Example 8

### Efficacy of the rAAVs for the treatment of MPS

Expression cassettes including a nucleotide sequence having at least 85% sequence identity to SEQ ID NO: 1 that codifies for the protein SEQ ID NO: 2 (a codon optimized version of the human sulfamidase cDNA sequence (co-hu-SFMD)) were designed and obtained. Codon optimization was performed to increase the efficiency of SFMD protein production in human beings by utilizing the most abundant tRNAs to the species and by also taking into account its particular translation profile. Mice were utilized for experimental purposes due to their similarity to human beings and the predictive capacity of the mouse animal model.

To ensure that this sequence led to the production of active sulfamidase, male MPSIIIA mice (MPS) were intravenously injected with 1x10¹² vg of an AAV9 vector in which co-hu-SFMD was expressed under the control of the ubiquitous CAG promoter. The activity of sulfamidase in the serum of these mice reached levels similar to that of healthy wild-type animals and was maintained for the duration of the study (2 months) (see FIG. 1A). This sustained sulfamidase activity led to the normalization of the GAG content in the livers of these animals, similar to what had been observed with the AAV9-delivered murine transgene (see FIG. 1 B).

All publications mentioned hereinabove are hereby incorporated in their entirety by reference. While the foregoing invention has been described in some detail for purposes of clarity and understanding, it will be appreciated by one skilled in the art from a reading of this disclosure that various changes in form and detail can be made without departing from the true scope of the invention and appended claims.

## Claims

1. A method for the production of recombinant adeno-associated viral vectors (rAAV), wherein said rAAVs comprise a nucleotide sequence having at least 85% sequence identity to SEQ ID NO: 1 that codifies for the protein SEQ ID NO: 2, wherein the method comprises the following steps:
(i) infecting an insect cell line with either 1, 2 or 3 baculovirus expression vectors comprising the *rep* and cap genes and a nucleotide sequence having at least 85% sequence identity to SEQ ID NO: 1 that codifies for the protein SEQ ID NO: 2 interposed between a first AAV terminal repeat and a second AAV terminal repeat, and
(ii) purifying the rAAVs produced by the cell culture of step (i).

2. The method according to claim 1 wherein the nucleotide sequence is SEQ ID NO: 1.

3. The method according to any one of claims 1 or 2, wherein the nucleotide sequence is operably linked to a CAG or a hAAT promoter.

4. The method according to any one of claims 1 to 3, wherein the nucleotide sequence is SEQ ID NO: 1 and it is operably linked to a CAG promoter.

5. The method according to any one of claims 1 to 3, wherein the sequence of the hAAT promoter is SEQ ID NO: 3.

6. The method according to any one of claims 1 to 5, wherein the insect cell line is Sf9 cell line.

7. The method according to any one of claims 1 to 6, wherein the insect cell line is stably transfected with the *rep* and cap genes.

8. The method according to claim 7 wherein the insect cell line is infected with one baculovirus expression vector comprising the nucleotide sequence having at least 85% sequence identity to SEQ ID NO: 1 that codifies for the protein SEQ ID NO: 2 interposed between a first AAV terminal repeat and a second AAV terminal repeat, and an inmediate-early 1 transcriptional transregulator.

9. The method according to any one of claims 1 to 6, wherein the insect cell line is infected with 2 baculovirus expression vectors, one comprising the *rep* and cap genes, and the second comprising the nucleotide sequence having at least 85% sequence identity to SEQ ID NO: 1 that codifies for the protein SEQ ID NO: 2 interposed between a first AAV terminal repeat and a second AAV terminal repeat.

10. The method according to any one of claims 1 to 6, wherein the insect cell line is infected with 3 baculovirus expression vectors, one comprising the rep genes, the second comprising the cap genes, and the third one comprising the nucleotide sequence having at least 85% sequence identity to SEQ ID NO: 1 that codifies for the protein SEQ ID NO: 2 interposed between a first AAV terminal repeat and a second AAV terminal repeat.

11. The method according to any one of claims 1 to 10, wherein the serotype of the rAAVs is 1, 2, 5, 7, 8 or 9.

12. The method according to claim 11, wherein the serotype of the rAAVs is 9.

13. The method according to any one of claims 1 to 12, wherein the rep genes are Rep78 and Rep52.

14. The method according to claim 13, wherein the expression of Rep78 is directed by an attenuated baculovirus promoter and/or the expression of Rep52 is directed by a strong late baculovirus promoter, preferably by p10.

15. The method according to any one of claims 1 to 14, wherein 10¹³ to 10¹⁵ particles of rAAV are obtained per liter of culture.


SEQUENCE LISTING

<110>  Laboratorios del Dr. Esteve
       Universitat Autónoma de Barcelona

<120>  METHODS FOR THE PRODUCTION OF VECTORS

<130>  EP2212.3

<160>  3

<170>  PatentIn version 3.5

<210>  1
<211>  1515
<212>  DNA
<213>  artificial sequence

<220>
<223>  codon-optimized sequence of human sulfamidase

<400>  1
gccaccatga gctgccctgt gcccgcctgt tgtgccctgc tgctggtgct gggactgtgc      60

agagccagac cccggaacgc tctgctgctg ctggccgacg atggcggatt tgagagcggc     120

gcctacaaca acagcgccat tgccacccct catctggacg ccctggccag aagaagcctg     180

ctgttccgga acgccttcac cagcgtgtcc agctgcagcc ctagcagagc ttccctgctg     240

acaggcctgc cccagcatca gaatggcatg tacggcctgc accaggatgt gcatcacttc     300

aacagcttcg acaaagtgcg gagcctgcca ctgctcctgt cacaggctgg cgtgagaacc     360

ggcatcatcg gcaagaaaca cgtgggcccc gagacagtgt accccttcga cttcgcctac     420

accgaagaga acggcagcgt gctgcaggtc ggccggaaca tcacccggat caagctgctc     480

gtgcggaagt ttctccagac ccaggacgac cggcccttct tcctgtacgt ggccttccac     540

gaccctcaca gatgcggcca cagccagccc cagtacggca ccttctgcga gaagttcggc     600

aacggcgaga gcggcatggg cagaatcccc gactggaccc cccaggcata cgaccctctg     660

gacgtgctgg tgccctactt cgtgcccaac acccctgccg ccagagctga tctggccgcc     720

cagtacacca ccgtgggcag aatggatcag ggcgtgggcc tggtgctgca ggaactgagg     780

gacgctggcg tgctgaacga caccctggtc atcttcacct ccgacaacgg catcccattc     840

cccagcggcc ggaccaatct gtactggccc ggcacagccg aacctctgct ggtgtccagc     900

cccgagcacc ctaagagatg gggccaggtg tccgaggcct acgtgtccct gctggacctg     960

acccccacca tcctggactg gttcagcatc ccctacccca gctacgccat ctttggaagc    1020

aagaccatcc acctgaccgg cagatctctg ctgcctgccc tggaagctga gcctctgtgg    1080

gccaccgtgt tcggcagcca gagccaccac gaagtgacca tgagctaccc catgcggagc    1140

gtgcagcacc ggcacttccg gctggtgcac aacctgaact tcaagatgcc cttcccaatc    1200

gaccaggact tttacgtgtc ccccaccttc caggacctgc tgaacagaac cacagccggc    1260

cagcccaccg gctggtacaa ggacctgcgg cactactact accgggccag atgggagctg    1320

tacgacagaa gccgggaccc ccacgagaca cagaacctgg ccaccgaccc cagattcgcc 1380

cagctcctgg aaatgctgcg ggaccagctg gccaagtggc agtgggagac acacgaccct 1440

tgggtctgcg ctcccgacgg cgtgctggaa gagaagctgt ccccccagtg ccagccactg 1500

cacaacgagc tgtga 1515

<210> 2
<211> 502
<212> PRT
<213> Homo sapiens

<400> 2

<210>  3
<211>  981
<212>  DNA
<213>  artificial sequence

<220>
<223>  liver-specific human alpha1-antitrypsin promoter

<400>  3
cagagaggtc tctgacctct gccccagctc caaggtcagc aggcagggag ggctgtgtgt        60

ttgctgtttg ctgcttgcaa tgtttgccca ttttagggac atgagtaggc tgaagtttgt       120

tcagtgtgga cttcagaggc agcacacaaa cagcaagctt gcgaattcca gtctacagag       180

aggtctctga cctctgcccc agctccaagg tcagcaggca gggagggctg tgtgtttgct       240

gtttgctgct tgcaatgttt gcccatttta gggacatgag taggctgaag tttgttcagt       300

gtggacttca gaggcagcac acaaacagca agcttgcgaa ttccagtcta cagagaggtc       360

tctgacctct gccccagctc caaggtcagc aggcagggag ggctgtgtgt ttgctgtttg       420

ctgcttgcaa tgtttgccca ttttagggac atgagtaggc tgaagtttgt tcagtgtgga       480

cttcagaggc agcacacaaa cagcaagctt tgctctagac tggaattcgt cgacgagctc       540

cctatagtga gtcgtattag aggccgactg acccggtacc cggggatctt gctaccagtg       600

gaacagccac taaggattct gcagtgagag cagagggcca gctaagtggt actctcccag       660

agactgtctg actcacgcca ccccctccac cttggacaca ggacgctgtg gtttctgagc       720

caggtacaat gactcctttc ggtaagtgca gtggaagctg tacactgccc aggcaaagcg       780

tccgggcagc gtaggcgggc gactcagatc ccagccagtg gacttagccc ctgtttgctc       840

ctccgataac tggggtgacc ttggttaata ttcaccagca gcctcccccg ttgcccctct       900

ggatccactg cttaaatacg gacgaggaca gggccctgtc tcctcagctt caggcaccac       960

cactgacctg ggacagtgaa t                                                 981